Europäisches Patentamt

European Patent Office

Office européen des brevets

(19)

(11) Publication number: **0 017 106**
**B1**

# EUROPEAN PATENT SPECIFICATION

(45) Date of publication of patent specification: **01.02.84**

(21) Application number: **80101506.6**

(22) Date of filing: **21.03.80**

(51) Int. Cl.³: **G 01 N 33/28,**
**G 01 N 35/08**

(54) Automatic analyzer for combustible gas in oil.

(30) Priority: **26.03.79 JP 35322/79**

(43) Date of publication of application:
**15.10.80 Bulletin 80/21**

(45) Publication of the grant of the patent:
**01.02.84 Bulletin 84/5**

(84) Designated Contracting States:
**DE FR GB**

(56) References cited:
**DE - A - 1 598 730**
**DE - A - 2 160 526**
**US - A - 2 987 912**
**US - A - 3 683 669**
**US - A - 3 844 160**
**US - A - 3 922 904**
**US - A - 3 942 792**

(73) Proprietor: **MITSUBISHI DENKI KABUSHIKI KAISHA**
**2-3, Marunouchi 2-chome Chiyoda-ku Tokyo 100 (JP)**

(73) Proprietor: **Yamada, Mitsuhiro**
**Kansai Electric Power Company Incorporated Sohgo Gijyutsu Kenkyusho No.1, Nakohji Ichinotsubo**
**Amagasaki-shi Hyogo-ken (JP)**

(73) Proprietor: **Katayama, Hirokazu**
**Kansai Electric Power Company Incorporated Sohgo Gijyutsu Kenkyusho No.1, Nakohji Ichinotsubo**
**Amagasaki-shi Hyogo-ken (JP)**

(72) Inventor: **Yamada, Mitsuhiro Kansai Electric Power Co., Inc.**
**Sohgo Gijyutsu Kenkyusho, No. 1,Nakohji**
**Ichinotsubo Amagasaki-shi,Hyogo-ken (JP)**
Inventor: **Katayama, Hirokazu Kansai Electric**
**Power Co., Inc.**
**Sohgo Gijyutsu Kenkyusho,No.1,Nakohji**
**Ichinotsubo**
**Amagasaki-shi,Hyogo-ken (JP)**
Inventor: **Ishii, Toshitsugu Mitsubishi Denki K.K.**
**Akoh Seisakusho, No.651,Tenwa**
**Akoh-shi,Hyogo-ken, (JP)**
Inventor: **Makino, Yoshihiro Mitsubishi Denki K.K.**
**Akoh Seisakusho, No.651,Tenwa**
**Akoh-shi,Hyogo-ken, (JP)**
Inventor: **Kamio, Masashi Mitsubishi Denki K.K.**
**Itami Seisakusho,No.80,Aza-Nakano**
**Minamishimizu Amagasaki-shi Hyogo-ken (JP)**

(74) Representative: **Wächtershäuser, Günter, Dr. et al,**
**Tal 29**
**D-8000 München 2 (DE)**

Automatic analyzer for combustible gas in oil

The present invention relates to a device for automatically analyzing the content of a combustible gas in an oil with an oil sampler for metering an oil sample into a vessel, with a combustible gas desorbing device for transferring a proportion of the combustible gas from the oil phase into a gas phase and with a gas discharging device for feeding the gas phase through a gas analyzer.

Such a device is known from US—A—3 844 160. In the prior art device the combustible gas is liberated from the oil phase by creating a partial vacuum in a cylinder as the combustible gas desorbing device. Therefore the only gas phase being available for the measurement consists of the desorbed gas. However, if the device is used for an early detection of a fault state of an electric apparatus by measuring the content of combustible gases, which have been formed within the oil by abnormal electrical phenomena, such as discharges, arcing, local overheating or the like, the content of the combustible gases at the very early stages of a fault state is very small. Consequently, the available gas volume is very small in the conventional device during early stages of a fault state. The handling of such small gas volumes is rather difficult. Further, with the conventional device, disadvantageously all process steps beginning with the metering of the oil and ending with the gas analyzation process must be completed until the next testing cycle may follow, since all process steps of metering an oil sample into a vessel, transferring a proportion of the combustible gas into a gas phase and feeding the gas phase into the gas analyzer are conducted in a single vessel. Therefore the requirement of a speedy detection of small concentrations of a gas cannot be sufficiently fulfilled by the device of US—A—3 844 160.

From US—A—2 987 912 an apparatus is known, which comprises a gas bubbler device for desorbing a dissolved evaporatable component from a liquid phase. However, this conventional device serves for repeatedly bubbling the air contained within the bubbling vessel through the liquid in a number of cycles in order to establish a partition equilibrium between the liquid phase and the gas phase. After the equilibrium has been established, which takes several minutes, the measurement of the gas component to be measured is effected. This process is necessarily quite time-consuming, and the process of measuring the gas components cannot be decoupled from the bubbling vessel so that the entire equipment is in operation throughout one measuring cycle. An increase of the efficiency and speed of the measuring system is only possible if several complete systems are multiplexed.

It is therefore an object of the present invention to modify a device according to the preamble of the main claim in such a way that a speedy detection of a concentration of a combustible gas dissolved in an oil may be conducted and even small quantities of gas may be detected reliably.

This problem is solved by a device according to the preamble of the main claim, characterized in that the combustible gas desorbing device comprises a gas bubbler for bubbling gas through the oil sample in said vessel and that the gas discharging device is adapted for intermediately receiving the charge of gas bubbled through the oil sample and for subsequently feeding said charge of gas through the gas analyzer while being disconnected from said vessel by a valve.

With this device, the combustible gas dissolved in oil can easily be sampled even though the quantity of the gas in the oil is small and the concentration of the combustible gas dissolved in oil can be measured at a constant time interval in a very speedy manner.

Brief Description of the Drawing

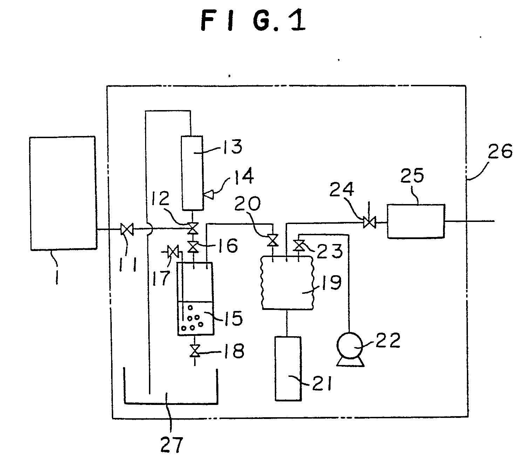

Figure 1 is a schematic view of one embodiment of an automatic analyzer for a combustible gas in an oil according to the present invention.

Detailed Description of the Preferred Embodiments

The present invention has been proposed to overcome the disadvantages of the conventional apparatus. The purpose of the present invention is to provide the automatic analyzer for the combustible gas dissolved in the oil which can operate speedily and can easily sample the gas even though the quantity of the gas dissolved in the oil is small. The automatic analyzer comprises the gas desorbing device wherein the sample of the oil is sampled into the vessel and the gas is fed into the vessel to desorb the dissolved gas dissolved in the oil; the gas discharger which is connected to the vessel to discharge the desorbed gas out of the vessel; and the combustible gas detector which detects the concentration of the combustible gas in the desorbed gas.

Referring to the drawing, one embodiment of the automatic analyzer for the combustible gas dissolved in the oil of the present invention will be described.

In Figure 1, the reference (11) designates an electromagnetic valve for sampling the sample oil from a transformer (1); (12) designates a three way electromagnetic valve which separates the flow of the sample oil sampled by the electromagnetic valve (11), into two ways by the switching operation. The reference numeral (13) designates an oil sampler, one end of which is connected to one port of the three-way electromagnetic valve (12) and the other end of which is connected to a waste oil vessel (27). The reference numeral (14) designates an oil level detector which is equipped with the sampled oil messcylinder (13); (15) designates a bubbling vessel which is connected through an electromagnetic valve (16) to another port of the three-way electromagnetic valve (12) and is connected through an electromagnetic valve (17) to the outside of the vessel and the bottom of the bubbling vessel is connected through an electromagnetic valve (18) to the waste oil vessel (27); (19) designates a bellows type gas discharger which is connected through an electromagnetic valve (20) to the bubbling vessel (15) and is driven by a bellows drivers (21) and is connected through an electromagnetic valve (23) to a vacuum pump (22); (24) designates a three way electromagnetic valve which is switched to connect to the bellows type gas discharger (19) and the combustible gas detector (25) or the bellows type gas discharger (19) and the atmosphere respectively; and (26) designates a case for holding these equipments in one unit.

The operation of the embodiment having the above-mentioned structure will be illustrated.

In the sampling of the oil from the transformer (1), the electromagnetic valves (17), (18) are closed and the electromagnetic valves (16), (20), (23) are opened. The three way electromagnetic valves (12), (24) are respectively connected to the transformer (1) and the oil sampler (13) and to the atmosphere and the combustible gas detector (25). The bellows type gas discharger (19) is extended to be the maximum length by the bellows driver (21) and the system is evacuated by the vacuum pump (22). When the system is evacuated, the electromagnetic valves (16), (23) are closed and the electromagnetic valve (11) is opened to feed the oil from the transformer into the oil sampler (13) to overflow the oil from the messcylinder (13) into the waste oil vessel (27) and then, electromagnetic valve (11) is closed. The three-way electromagnetic valve (12) is connected to the oil sampler (13) and the bubbling vessel (15). The electromagnetic valve (16) is opened so that the oil flow down as the sample into the bubbling vessel (15). When the level of the oil in the oil sampler (13) is decreased to the oil level detector (14), the electromagnetic valve (16) is closed. When the electromagnetic valve (17) is opened, air is fed into the oil and is further fed into the bellows type gas discharger (19) together with the extracted gas dissolved in the oil. The movement of air is continued until the pressure in the bellows type gas discharger (19) reaches atmospheric pressure. When it reaches atmospheric pressure, the electromagnetic valve (20) is closed and the electromagnetic valve (18) is opened. The current is fed to the combustible gas detector (25) and clean air is sucked from the atmosphere through the three way electromagnetic valve (23) by a pump (not shown) and the zero level of the combustible gas detector (25) is adjusted, and then, the combustible gas detector (25) is connected through the three way electromagnetic valve (24) to the bellows type gas discharger (19) and simultaneously, the electromagnetic valve (23) is opened and the bellows gas discharger (19) is contracted at a constant velocity by the bellows drivers (21) to feed the sample gas into the combustible gas detector (25) so as to measure the concentration of the combustible gas.

In this embodiment, the insulating oil in the transformer (1) is used as the sample oil. It is not limited to the use of insulating oil. Air is fed into the bubbling vessel (15) to operate the bubbling. The similar effect is attained by using an inert gas for the bubbling. It is also possible to use a piston type, pressurizing pump type and vacuum pump type discharger instead of the bellows type gas discharger (19).

As stated above, in accordance with the present invention, the automatic analyzer for a combustible gas comprises the gas desorbing device wherein the oil is sampled in the vessel and the gas is fed into the vessel to desorb the dissolved gas dissolved in the oil; a gas discharger which is connected to the vessel to discharge the desorbed gas out of the vessel; and the combustible gas detector which detects the concentration of the combustible gas in the desorbed gas whereby the operation can be carried out speedily and the combustible gas can be easily sampled, even though the quantity of the gas in the oil is small. The advantages in practical use are remarkable.

**Claims**

1. A device for automatically analyzing the content of a combustible gas in an oil with an oil sampler (13) for metering an oil sample into a vessel (15), with a combustible gas desorbing device for transferring a proportion of the combustible gas from the oil phase into a gas phase and with a gas discharging device (19, 21) for feeding the gas phase through a gas analyzer (25), characterized in that the combustible gas desorbing device comprises a gas bubbler for bubbling gas through the oil sample in said vessel (15) and that the gas discharging device (19, 21) is adapted for intermediately receiving the charge of gas bubbled through the oil sample and for subsequently feeding said charge of gas through the gas analyzer (25) while being disconnected from said vessel by a valve (20).

2. A device according to claim 1, characterized in that said oil is an insulating oil in a transformer.

3. A device according to claim 1, characterized in that the gas being bubbled is air.

4. A device according to claim 1, characterized in that the gas discharging device (19, 21) comprises a bellows type gas discharger (19).

5. A device according to claim 1, characterized in that the combustible gas desorbing device, the gas discharging device (19, 21) and the gas analyzer (25) are held in one case (26).

## Revendications

1. Dispositif pour analyser automatiquement la teneur d'un gaz combustible dans une huile, comprenant un échantillonneur d'huile (13) pour mesurer un échantillon d'huile dans un récipient (15), un dispositif de désorption du gaz combustible pour transférer une partie du gaz combustible de la phase d'huile dans une phase gazeuse et un dispositif de décharge de gaz (19, 21) pour faire passer la phase gazeuse dans un analyseur de gaz (25), caractérisé en ce que le dispositif de désorption du gaz combustible comprend un barboteur pour le barbotage du gaz à travers l'échantillon d'huile dans ledit récipient (15), et en ce que le dispositif de décharge du gaz (19, 21) est adapté pour recevoir de façon intermédiaire la charge de gaz résultant du barbotage à travers l'échantillon d'huile et pour ensuite faire passer cette charge de gaz à travers l'analyseur de gaz (25), pendant qu'il est déconnecté dudit récipient par une valve (20).

2. Dispositif selon la revendication 1, caractérisé en ce que l'huile est une huile isolante dans un transformateur.

3. Dispositif selon la revendication 1, caractérisé en ce que le gaz de barbotage est l'air.

4. Dispositif selon la revendication 1, caractérisé en ce que le dispositif de décharge du gaz (19, 21) est un déchargeur de gaz du type à soufflet (19).

5. Dispositif selon la revendication 1, caractérisé en ce que le dispositif de désorption du gaz combustible, le dispositif de décharge du gaz (19, 21) et l'analyseur de gaz (25) sont réunis dans un seul boîtier (26).

## Patentansprüche

1. Vorrichtung zur automatischen Bestimmung des Gehalts eines brennbaren Gases in einem Öl mit einem Ölprobennehmer (13) zum Einmessen einer Ölprobe in ein Gefäß (15), mit einer brennbares Gas desorbierenden Vorrichtung zur Überführung eines Anteils des brennbaren Gases aus der Ölphase in eine Gasphase und mit einer Gasablaßvorrichtung (19, 21) zum Einspeisen der Gasphase durch einen Gasanalysator (25), dadurch gekennzeichnet, daß die brennbares Gas desorbierende Vorrichtung eine Gassprudeleinrichtung zum Durchsprudeln von Gas durch die Ölprobe in dem Gefäß (15) umfaßt und daß die Gasablaßvorrichtung (19, 21) eingerichtet ist zur zwischenzeitlichen Aufnahme der durch die Ölprobe durchgesprudeltem Gascharge und zum nachfolgenden Einspeisen der Gascharge durch den Gasanalysator (25), während die Verbindung zu dem Gefäß durch ein Ventil (20) unterbrochen ist.

2. Vorrichtung nach Anspruch 1, dadurch gekennzeichnet, daß das Öl ein Isolieröl in einem Transformator ist.

3. Vorrichtung nach Anspruch 1, dadurch gekennzeichnet, daß das eingesprudelte Gas Luft ist.

4. Vorrichtung nach Anspruch 1, dadurch gekennzeichnet, daß die Gasablaßvorrichtung (19, 21) eine Gasablaßeinrichtung (19) vom Balgtyp umfaßt.

5. Vorrichtung nach Anspruch 1, dadurch gekennzeichnet, daß die brennbares Gas desorbierende Vorrichtung, die Gasablaßvorrichtung (19, 21) und der Gasanalysator (25) in einem einzigen Gehäuse (26) angeordnet sind.

# F I G. 1